(19)

Europäisches Patentamt
European Patent Office
Office européen des brevets

(11) **EP 3 522 171 A1**

(12) **EUROPEAN PATENT APPLICATION**

(43) Date of publication:
**07.08.2019 Bulletin 2019/32**

(51) Int Cl.:
**G16H 50/30** (2018.01)

(21) Application number: **18197414.8**

(22) Date of filing: **28.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.02.2018 EP 18155011**

(71) Applicants:
• **Université de Rennes 1**
**35000 Rennes (FR)**

• **Institut National de la Santé et de la Recherche Médicale**
**75013 Paris (FR)**

(72) Inventors:
• **HASSAN, Mahmoud**
**35042 RENNES (FR)**
• **WENDLING, Fabrice**
**35042 RENNES (FR)**
• **KABBARA, Aya**
**35042 RENNES (FR)**

(74) Representative: **Vidon Brevets & Stratégie**
**16B, rue de Jouanet**
**BP 90333**
**35703 Rennes Cedex 7 (FR)**

(54) **METHOD, DEVICE AND PROGRAM FOR DETERMINING A DISEASE SCORE**

(57) The invention relates to a method of constructing a value representative of an interaction between a plurality of brain regions, the method being implemented by an electronic device, said electronic device comprising a processor and a memory. According to the invention, the method comprises:
- obtaining (10), in the form of connectivity matrices, dynamic functional networks, which are representative of electrical signals measured for a predetermined number of points of interest, called nodes, within a cerebral cortex during a given time period;
- determining (20), from at least one of said connectivity matrices, a global efficiency (GE) score and at least one clustering coefficient score (Cc) for each node of said at least one of said connectivity matrices;
- calculating (30) a value representative of an interaction between the plurality of brain networks in the form of a distribution ratio (DI) using said global efficiency score and said clustering coefficient scores (Cc).

Figure 3

**Description**

**1. Field of the disclosure**

Worldwide, about 35 million people are estimated to have dementia (World Health Organization 2012). Alzheimer's disease (AD), the most common cause of dementia, is a neurological disorder essentially characterized by progressive impairment of memory and other cognitive functions. Emerging evidence show that the progressive evolution in AD is related to pathological changes in large-scale neural networks. Therefore, from a clinical perspective, the demand is high for non-invasive and easy- to-use methods to identify these pathological networks. The invention relates to the processing of a novel network-based *'measure'* able to characterize network alterations and associated cognitive deficits in AD patients.

**2. Background**

The present section is intended to introduce the reader to various aspects of art, which may be related to various aspects of the present disclosure that are described and/or claimed below. This discussion is believed to be helpful in providing the reader with background information to facilitate a better understanding of the various aspects of the present disclosure. Accordingly, it should be understood that these statements are to be read in this light, and not as admissions of prior art.

In the context of developing *'neuromarker'* for cognitive deficits from neuroimaging techniques, Electroencephalography (EEG) has some major assets since it is a non-invasive, easy to use and clinically available technique. A potential framework for advanced EEG analysis is the emerging technique called "MEG/EEG source connectivity". In addition, and as shown by several recent studies (Hassan *et al.,* 2016*,* 2017), this method could indeed provide some responses to clinical demand, provided that appropriate information processing is performed. Previous results, using the EEG source connectivity methods, showed alterations in the functional connectivity at the theta and alpha2 bands in AD patients compared to controls. Relationships between the dysfunctional connections in AD patients and the cognitive decline progression were also observed.

However, to what extent the AD modifies the brain network segregation (local information processing) and integration (global information processing) remains unclear.

**3. Summary**

An object of the proposed technique is to process a neuromarker, based on EEG measurement, which allows defining a ratio between local information processing and global information processing. More precisely, the inventors raised one main question: i) is there a correlation between the network disruptions in term of segregation/integration and the cognitive score of the AD patients? To tackle this issue, the inventors combined the use of the EEG source connectivity with the graph theory-based analysis. Resting state EEG data were recorded from 20 participants (10 AD patients and 10 age-matched controls). The functional networks are reconstructed at the cortical level from scalp EEG electrodes.

According to an aspect of the present disclosure, it is disclosed a method of constructing a value representative of an interaction between a plurality of brain regions, the method being implemented by an electronic device, said electronic device comprising a processor and a memory, characterized in that it comprises:

- obtaining, in the form of connectivity matrices, dynamic functional networks, which are representative of electrical signals measured for a predetermined number of points of interest, called nodes, within a cerebral cortex during a given time period;
- determining, from at least one of said connectivity matrices, a global efficiency (GE) score and at least one clustering coefficient score (Cc) for each node of said at least one of said connectivity matrices;
- calculating a value representative of an interaction between the plurality of brain networks in the form of a distribution ratio using said global efficiency score and said clustering coefficient scores (Cc).

Thus, the invention allows to simply gives information about the potential status of a patient regarding AD, which may be used in further process so as to confirm or eliminate the need of other costly physical examination by a doctor or a practitioner.

According to a specific feature, determining said global efficiency (GE) score comprises calculating:

$$GE = \frac{1}{N} \sum_{i}^{N} E_i$$

Where $E_i$ is the efficiency of each node I computed through the shortest path lengths between nodes.

According to a specific feature, determining one clustering coefficient score (Cc) of one node comprises calculating:

$$Cc(i) = \frac{2L_i}{k_i(k_i - 1)}$$

Where L represents the number of links between the $k_i$ neighbors of node i.

According to a specific feature, calculating comprises, for at least one connectivity matrix associated to at least one dynamic functional network:

$$DI = \frac{GE}{\sum_{i}^{N} Cc}$$

where GE is the global efficiency of the network, Cc is the clustering coefficient and N is the number of nodes in the network.

According to a specific feature, N is equal to 68 and corresponds to a given number of regions of the brain which may be easily obtained for any patient.

According to a specific feature, obtaining connectivity matrices comprises:

- obtaining signals representing of a cerebral activity for a given period of time;
- constructing, using the previously obtained signals, a plurality of data structures representative of the functional connectivity between a plurality of regions of interest for a given frequency;
- identifying, within said plurality of functional connectivity data structures, dynamic functional networks;

According to a specific feature, determining comprises, for a given connectivity matrix:

- calculating the global efficiency (GE) score;
- calculating an individual clustering coefficient score (Cc) for each node of said connectivity matrix.

In another embodiment, the disclosure also relates to an electronic device for obtaining a value representative of an interaction between a plurality of brain networks, the electronic device comprising a processor and a memory. According to the disclosure, the device comprises the necessary means for:

- obtaining, in the form of connectivity matrices, dynamic functional networks, which are representative of electrical signals measured for a predetermined number of points of interest, called nodes, within a cerebral cortex during a given time period;
- determining, from at least one of said connectivity matrices, a global efficiency (GE) score and at least one clustering coefficient score (Cc) for each node of said at least one of said connectivity matrices;
- calculating a value representative of an interaction between the plurality of brain networks in the form of a distribution ratio using said global efficiency score and said clustering coefficient scores (Cc).

According to one specific implementation, the different steps of the method according to the invention are implemented by one or more software programs or computer programs comprising software instructions that are to be executed by a processor of an information-processing device, such as a terminal according to the invention and being designed to command the execution of the different steps of the methods.

The invention is therefore also aimed at providing a computer program, capable of being executed by a computer or by a data processor, this program comprising instructions to command the execution of the steps of a method as mentioned here above.

This program can use any programming language whatsoever and be in the form of source code, object code or intermediate code between source code and object code such as in a partially compiled form or in any other desirable form whatsoever.

The invention is also aimed at providing an information carrier readable by a data processor and comprising instructions of a program as mentioned here above.

The information carrier can be any entity or communications terminal whatsoever capable of storing the program. For example, the carrier can comprise a storage means such as a ROM, for example, a CD ROM or microelectronic circuit ROM or again a magnetic recording means, for example a floppy disk or a hard disk drive.

Furthermore, the information carrier can be a transmissible carrier such as an electrical or optical signal that can be conveyed via an electrical or optical cable, by radio or by other means. The program according to the proposed technique can especially be uploaded to an Internet type network.

As an alternative, the information carrier can be an integrated circuit into which the program is incorporated, the circuit being adapted to executing or to being used in the execution of the method in question.

According to one embodiment, the proposed technique is implemented by means of software and/or hardware components. In this respect, the term "module" can correspond in this document equally well to a software component and to a hardware component or to a set of hardware and software components.

A software component corresponds to one or more software module programs, one or more subprograms of a program or more generally to any element of a program or a piece of software capable of implementing a function or a set of functions according to what is described here below for the module concerned. Such a software component is executed by a data processor of a physical entity (terminal, server, gateway, router etc) and is capable of accessing hardware resources of this physical entity (memories, recording media, communications buses, input/output electronic boards, user interfaces etc)

In the same way, a hardware component corresponds to any element of a hardware assembly capable of implementing a function or a set of functions according to what is described here below for the module concerned. It can be a programmable hardware component or a component with an integrated processor for the execution of software, for example, an integrated circuit, a smart card, a memory card, an electronic board for the execution of firmware etc.

Each component of the system described here above can of course implement its own software modules.

The different embodiments mentioned here above can be combined with one another to implement the proposed technique.

## 4. Figures

Explanations of the present disclosure can be better understood with reference to the following description and drawings, given by way of example and not limiting the scope of protection, and in which:

- figure 1 describes the full pipeline of treatment of the data according to an embodiment;
- figure 2 describes the relationship between the distribution ratio as disclosed and the MMSE score;
- figure 3 illustrates the mains steps of the process as disclosed;
- figure 4 disclose a simplified structure of a device of implementation of the process as disclosed.

## 5. Description

### 5.1. Principles

According to the invention, it is proposed a technique in which brain networks are build from EEG recordings (the techniques for achieving this reconstruction of networks are known and are not part of the invention in itself). However, the networks (obtained from sources reconstruction method) are processed in a new and inventive way so as to provide a distinction between the information which is locally process in given areas of the brain (segregation) and the information which processed globally, between several areas (integration). For achieving these results, the inventors had the idea to use some mathematical tools, and more specifically some topological tools for mapping the functioning of the processing of the information in the brain with the ways the topological tools describe the functioning of networks.

The inventors used EEG connectivity at the source level in AD patients. The inventors showed that AD networks are characterized by a reduction in their global performance (integration) associated with an enhancement in their local performance (segregation). The inventors showed also that these network topologies are correlated with the patient's cognitive scores. The inventors speculate that their processing method could contribute to the development of EEG-based test that could consolidate results of currently used neurophysiological tests.

The proposed method is included in the following general phases, which are more precisely described herein after:

- data acquisition and preprocessing;

- brain networks construction using the EEG source-connectivity method;
- network measures for identifying topological properties of the networks;
- statistical tests;

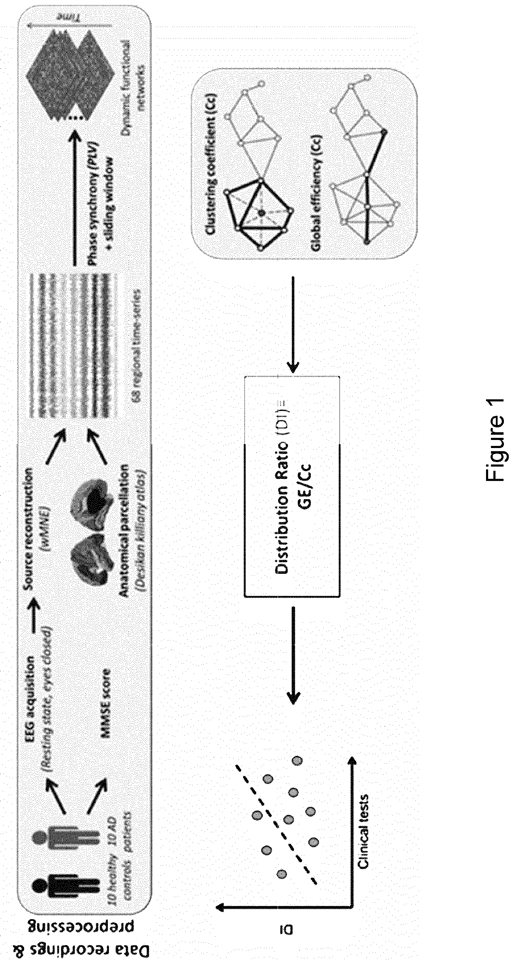

Figure 1 illustrate the *Structure of the process.* Data are recorded from 10 healthy controls and 10 AD patients during resting state paradigm (eyes closed). The cognitive performance was evaluated using MMSE score. The cortical sources are reconstructed using weighted minimum norm estimate (wMNE) inverse solution. Desikan Killiany atlas was used to anatomically parcellate the brain into 68 ROIs. The dynamic functional networks are then computed using phase synchrony method combined with a sliding window approach. In order to analyze the difference between healthy and AD networks, graph measures are extracted: clustering coefficient and global efficiency.

According to the invention, once the networks are reconstructed from the EEG source connectivity method, the nodes which compose these networks are challenged so as to obtain a score which allows specifying the connection of the nodes with each other in the network. Each node is then characterized as a function of several measurements and calculations made on the connections of a node with other node. On the basis of the previous results, segregation and integration are calculated so as to provide a distribution ratio.

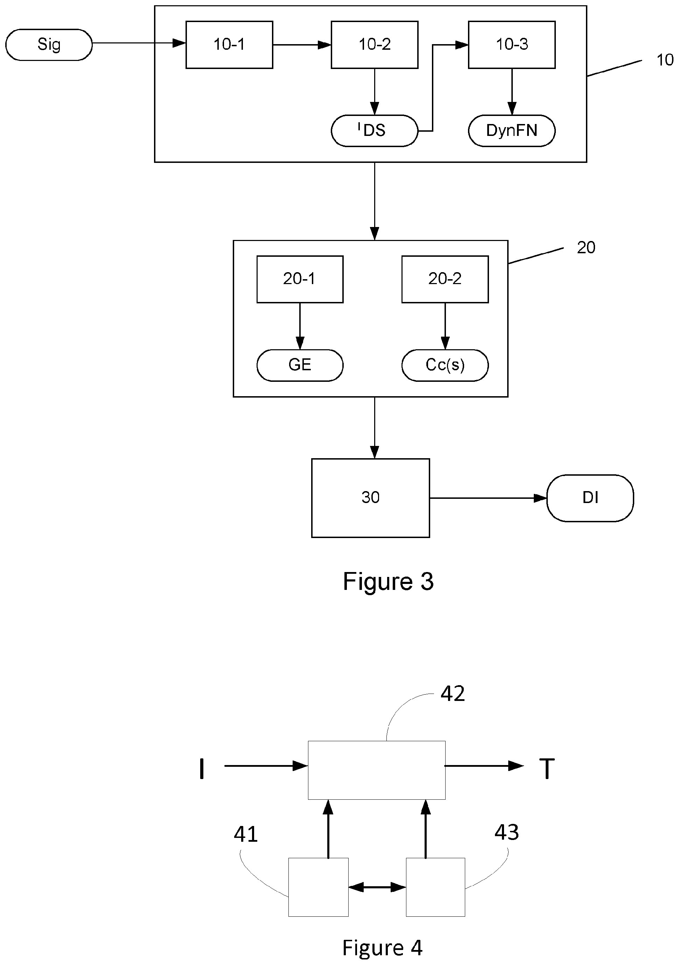

More specifically, in relation with figure 3, it is disclosed a method of obtaining a value representative of an interaction between a plurality of brain networks, the method being implemented by an electronic device, said electronic device comprising a processor and a memory. The method comprises:

- obtaining (10), in the form of connectivity matrices, dynamic functional networks, which are representative of electrical signals measured for a predetermined number of points of interest, called nodes, within a cerebral cortex during a given time period; this step of obtaining globally uses the EEG source connectivity method. The dynamic functional networks represent an electric activity between a predefined number of regions of interests (68) in the brain.
- determining (20), from at least one of said connectivity matrices (average over all dynamic matrices for each subject), a global efficiency (GE) score and at least one clustering coefficient score (Cc) for each node of said at least one of said connectivity matrices;
- calculating (30) a value representative of an interaction between the plurality of brain networks in the form of a distribution ratio using said global efficiency score and said clustering coefficient scores (Cc).

Segregation and integration are network measures, some of which being presented herein after in the disclosure.

According to the invention, it is thus possible to obtain an evaluation of the way the information is processed in the brain by comparing the local information processing intensity versus the global information processing intensity. These two results may allow confirming a psychophysiological evaluation made independently.

More specifically, obtaining (10) connectivity matrices comprises:

- obtaining (10-1) signals (Sig) representative of a cerebral activity for a given period of time;
- constructing (10-2), using the previously obtained signals, a plurality of data structures (DS) representative of the functional connectivity between a plurality of regions of interest for a given frequency;
- identifying (10-3), within said plurality of functional connectivity data structures, dynamic functional networks (DynFN) which are also called connectivity matrices;

Additionally; determining (20) comprises, for a given connectivity matrix:

- calculating (20-1) the global efficiency (GE) score;
- calculating (20-2) an individual clustering coefficient score (Cc) for each node of said connectivity matrix.

*5.2. Description of an embodiment*

*5.2.1. Materials and methods*

The pipeline of the process is illustrated in Figure 1, already presented.

5.2.1.1. Participants

This step is optional. The sole purpose is to obtain data which can be compared. Ten healthy controls (6 males and 4 females, age 64- 78 y) and ten patients diagnosed with AD (5 females and 5 males, age 66-81 y) participated in this study. All subjects provided informed consent in accordance with the local institutional review boards guidelines (CE-EDST-3-2017). Patients were recruited from the memory clinic of Dar al-Ajaza Hospital and from Mazloum Hospital, Tripoli, Lebanon. Age-matched healthy controls were recruited from Dar Al-Ajaza Hospital and the local community. For each subject medical history, a cognitive screening test and EEG acquisition were available. The mini-mental state examination (MMSE) was used as an indicator of the global cognitive performance. This test was widely used to characterize the overall cognitive level of AD patients and to estimate the severity and progression of cognitive impairment. Any score greater than or equal to 24 points out of 30 (MMSE $\geq$ 0.8) indicates normal cognitive functions. Below this score indicate cognitive impairment.

5.2.1.2. Data acquisition and preprocessing:

This step is optional in view of the process of obtaining the distribution ratio. In the case of one individual, this step can be done in a complete decorrelation of the process of obtaining the distribution ratio. One just need, for the obtention of the distribution ratio, to obtain lead field matrices from EEG records which could have been made previously.

EEG signals are recorded using a 32-channel EEG system (Twente Medical Systems International -TMSi-, Porti system) placed on the head according to the 10-20 system. Signals are sampled at 500 Hz and band-pass filtered between 0.1-45 Hz. All subjects underwent 10 min of resting-state in which they are asked to relax and keep their eyes closed without falling asleep.

The EEG signals are often contaminated by several sources of noises/artifacts. In order to pre-process these noisy-signals, the inventors followed the same steps used in several previous studies dealing with EEG resting state data. Briefly, the bad electrodes are first identified (i.e electrodes that are either completely flat or are contaminated by movements artifacts) by visual inspection. When needed, the power spectral density of electrodes was examined. Then, the bad channels are interpolated using the spherical approach implemented in EEGLAB. In addition, epochs with voltage fluctuation >+80 $\mu$V and <-80 $\mu$V are removed. Consequently, for each participant, four artifact-free epochs of 40s lengths are selected. This epoch length was largely used previously and considered as a good compromise between the needed temporal resolution and the reproducibility of the results. As the recorded EEG data used here have a very high temporal resolution ($\sim$1ms), the available samples are largely sufficient to compute consistent functional networks. By using a sliding window approach while calculating the functional connectivity, a high number of networks (different from a frequency band to another) are obtained for each 40s-epoch.

The EEGs and MRI template (ICBM152) are co-registered after identifying the anatomical landmarks (left and right pre-auricular points and nasion) using Brainstorm. An atlas-based segmentation approach was used to project EEGs onto an anatomical framework consisting of 68 cortical regions identified by means of Desikan-Killiany atlas. The lead field matrix is then computed for a cortical mesh of 15000 vertices using Open-MEEG.

5.2.1.3. Brain networks construction:

Brain networks are constructed using the "EEG source connectivity" method. It includes two main steps: 1) Reconstruct the temporal dynamics of the cortical sources by solving the inverse problem *(this is done from lead field matrices obtained from EEG records),* and 2) Measure the functional connectivity between the reconstructed time series. Here, the inventors used the weighted minimum norm estimate (wMNE) algorithm as inverse solution. The reconstructed regional time series are filtered in different frequency bands [theta (4-8 Hz); alpha1 (8-10 Hz); alpha2 (10-13 Hz); beta (13-30 Hz)]. The functional connectivity are computed, at each frequency band, between the regional time series using the phase locking value (PLV) measure. The PLV ranges between 0 (no phase locking) and 1 (full synchronization).

Using PLV, dynamic functional connectivity matrices are computed for each epoch using a sliding window technique. It consists in moving a time window of certain size $\delta$ along the time dimension of the epoch, and then PLV is calculated within each window. The inventors chose the smallest window length that is equal to

$$\frac{6}{central\ frequency}$$ where 6 is the number of 'cycles' at the given frequency band. In theta band, as the central frequency equals to 6 Hz, $\delta$ equals Is. Likewise, $\delta$ =666 ms in alpha1 band, 521 ms in alpha2 band, and 279 ms in beta band. Functional connectivity matrices are represented as graphs (i.e networks) composed of nodes, represented by the 68 ROIs, and edges corresponding to the functional connectivity assessed between the 68 regions. Thus, functional connectivity matrices have 68x68 dimension.

Considered $\delta$ values yield, for each epoch, we have 33 networks in theta band, 66 networks in alpha1 band, 76 networks in alpha2 band and 130 networks in beta band. In other words, for each epoch, 33 functional connectivity matrices are obtained in theta band, 66 functional connectivity matrices in alpha1 band, 76 functional connectivity matrices in alpha2 band and 130 functional connectivity matrices in beta band.

### 5.2.1.4. Network measures

The topological properties of identified networks are characterized using the following graph measures:

*Average clustering coefficient (Cc):* The clustering coefficient of a node represents how close its neighbors tend to cluster together. Accordingly, the average clustering coefficient of a network is considered as a direct measure of its segregation (i.e the degree to which a network is organized into local specialized regions). In brief, the clustering coefficient of a node is defined as the proportion of connections among its neighbors, divided by the number of connections that could possibly exist between them. For a given node i (brain region) in a graph G (with N nodes) connected to k edges, Cc is defined as:

$$Cc(i) = \frac{2L_i}{k_i(k_i - 1)}$$

Where L represents the number of links between the $k_i$ neighbors of node i.

*Global efficiency (GE):* The global efficiency of a network is the average inverse shortest path length. A short path length indicates that, on average, each node can reach other nodes with a path composed of only a few edges. Thus, the global efficiency is one of the most elementary indicators of network's integration (i.e the degree to which a network can share information between distributed regions). According to the disclosure, Global Efficiency (GE) is used as the integration factor of the distribution ratio. GE is defined as

$$GE = \frac{1}{N} \sum_i^N E_i$$

Where $E_i$ is the efficiency of each node I computed through the shortest path lengths (distance) between nodes N.

### 5.2.1.5. Statistical tests

To quantify the differences between healthy and AD networks in terms of clustering coefficient and global efficiency (integration/segregation measures) statistical tests are performed. For each subject, the inventors averaged all the metrics values obtained from the different networks among all epochs and time windows for each subject. As data are not normally distributed, the inventors assessed the statistical difference between the two groups using the Mann Whitney U Test also known as Rank-Sum Wilcoxon test (degree of freedom=18).

To deal with the family-wise error rate, the statistical tests are corrected for multiple comparisons using Bonferroni method

$$p_{Bonferroni\ adjusted} < \frac{0.05}{N}$$

with N (68) denotes the number of brain regions.

### 5.2.1.6. Calculation of the distribution ratio

Based on the results of the previous calculation, the distribution ratio is calculated.

The distribution ratio is based on the ratio between the network global connectivity (network integration) and the local connectivity (network segragation). For each patient, the new metric, called DI: Distribution Ratio, is defined as:

$$DI = \frac{GE}{\sum_i^N Cc}$$

where GE is the global efficiency of the network, Cc is the clustering coefficient and N is the number of nodes in the network.

*5.2.2. Results*

5.2.2.1. Network integration and segregation

Here, the inventors explored the difference of brain network dynamics between the two groups in terms of segregation using clustering coefficient and integration using the global efficiency measures. No group difference was observed in alpha1, alpha2 and beta bands. In theta band, an increase in clustering coefficient *(p=0.006; U=9, r=0.57)* associated with a decrease in global efficiency *(p=0.03; U=16, r=0.49)* was found in AD networks.

5.2.2.2. Correlation between network measures and cognitive scores

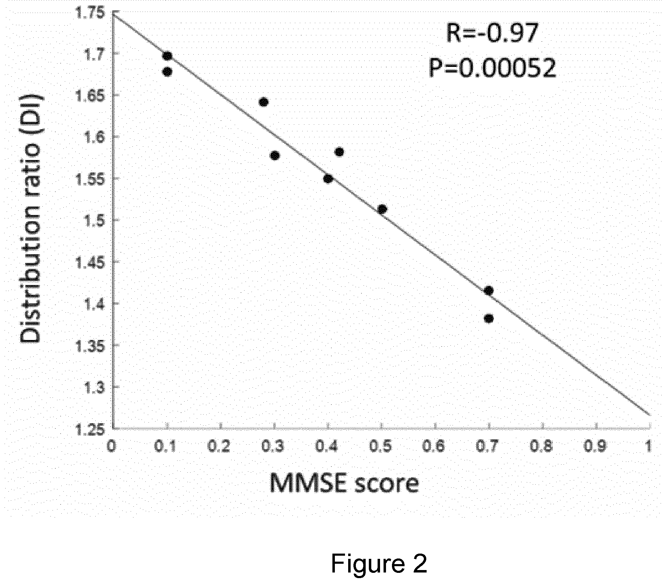

As exposed in figure 2, the distribution ratio shows an excellent correlation with the MMSE score (a cognitive score used to classify AD patients), with a negative correlation of r=-0.97 and p=0.00052, figure 2. The negative correlation indicates that the global connectivity (integration) decreases with the worsening of the disease

*5.2.3. Discussion*

The main objective in this study is to explore the topological properties of AD networks compared to healthy controls. Particularly, the inventors focused on examining the shifting balance between brain network integration and segregation in Alzheimer's disease. For this end, resting state EEG signals are recorded from 20 participants (10 AD patients and 10 controls). The cortical functional networks are reconstructed from scalp signals using the EEG source connectivity method. A sliding window approach was used to track the dynamics of networks. To examine the differences between the two groups (AD vs. controls), several network measures are extracted. The measure used to quantify the integration of networks is: the network global efficiency. To measure the segregation, the inventors extracted the clustering coefficient. Generally speaking, results showed that AD networks tend to have improved segregation (higher local information processing) and reduced integration (lower global information processing). Results showed also correlations between patients' cognitive performance (measured by the MMSE score) and network measures.

5.2.3.1. AD networks: high segregation and low integration

Results indicate that AD networks are characterized by lower integration (revealed by a decrease in the network global efficiency), and higher segregation (revealed by an increase in clustering coefficient,) compared to healthy control networks. One possible interpretation of the increased local connectivity is a possible compensatory mechanism that is triggered by the dysfunctional integration in the AD brain networks. These findings are in line with studies that revealed decrease in the network global efficiency and the participation coefficient in AD networks.

5.2.3.2. EEG Frequency bands

EEG is increasingly used to detect cognitive deficits in neurodegenerative disorders. One of the main and consistent findings is the shift to lower frequencies in Alzheimer's disease, using resting-state recordings. A slowing of EEGs in the theta power was also observed in Alzheimer's disease at early stage of the disease. Several previous studies have confirmed the importance of the theta band with regards to cognition. In addition, the importance of theta activity in controlling the working memory processes are widely reported. The inventor's results are in harmony with most of these studies. A potential interpretation of these findings is that disruption of low frequencies such as theta rhythms is due to degeneration phenomena of the of the attentional system.
Compared to other frequency bands, here the inventors found significant differences in theta band network characteristics in AD networks, namely, lower integration (low global efficiency), higher segregation (high average clustering). Using brain network analysis, several previous studies have observed alterations in the lower frequency bands in demented patients. These findings revealed loss in hubs, disruption in functional connectivity, reduction in network efficiency and a decrease in local integration in the alpha2 band.
Results also depict an opposite influence of the low frequency bands (theta, alpha1, alpha2) on the balance of integration/segregation compared to the higher frequency band (beta). A possible explanation is the complementary role of frequencies in conducting long/short range connections. In fact, while integrated information is mediated by low frequency bands, local information processing is mediated by high frequency bands.

5.2.3.3. Correlation between network measures and AD patient's cognitive scores

Single-subject analyses showed significant correlation between the MMSE score (used here to provide an overall measure of cognitive impairment) and network global efficiency and average clustering coefficient. Although the MMSE test has received high acceptance as a diagnostic test among researchers, it is recommended not to be used as a stand-alone single administration test. Previous studies have shown that age, education and socio-cultural variables affect the effectiveness of MMSE to detect cognitive impairment. Hence, it is more useful to include other tests that provide higher detection accuracy, as well as more specific scores (semantic, memory related... etc.). In addition, using a cognitive task that stimulates the affected networks in the case of AD (the memory network for instance) may improve the correlations with network-based metrics. It is worth noting that the MMSE is not the unique test for AD diagnosis. It is currently used within a set of other tests including physical exam (such as reflexes, muscle tone, balance) and brain imaging (such MRI and CT scan) aimed to pinpoint visible abnormalities related to conditions other than AD (stroke, trauma. etc.). However, when MRI is negative (no visible anatomical damages), the screening of cognitive performance using clinical tests such as MMSE (or other specific cognitive scores) are mandatory. Therefore, the proposed network-based metrics can be additional factors that neurologist needs to provide complete diagnosis.

*5.3. Devices and computer programs*

The invention also relates to an electronic device for the processing of data such as exposed herein before. The device comprises means and processing resources for implementing the method proposed.

According to a preferred implementation, the different steps of the methods of the invention are implemented by one or more software programs or computer program comprising software instructions to be executed by a data processor of a relay module according to the invention and designed to command the execution of different steps of the methods.

The invention is therefore also aimed at providing a program, capable of being executed by a computer or by a data processor, this program comprising instructions to command the execution of the steps of a method as mentioned here above.

This program can use any programming language whatsoever and be in the form of source code, object code or intermediate code between source code and object code such as in a partially compiled form or in any other desirable form whatsoever.

The invention is also aimed at providing an information carrier readable by a data processor and comprising instructions of a program as mentioned here above.

The information carrier can be any entity or communications terminal whatsoever capable of storing the program. For example, the carrier can comprise a storage means such as a ROM, for example, a CD ROM or microelectronic circuit ROM or again a magnetic recording means, for example a floppy disk or a hard disk drive.

Furthermore, the information carrier can be a transmissible carrier such as an electrical or optical signal that can be conveyed via an electrical or optical cable, by radio or by other means. The program according to the proposed technique can especially be uploaded to an Internet type network.

As an alternative, the information carrier can be an integrated circuit into which the program is incorporated, the circuit being adapted to executing or to being used in the execution of the method in question.

According to one embodiment, the proposed technique is implemented by means of software and/or hardware components. In this respect, the term "module" can correspond in this document equally well to a software component and to a hardware component or to a set of hardware and software components.

A software component corresponds to one or more software module programs, one or more subprograms of a program or more generally to any element of a program or a piece of software capable of implementing a function or a set of functions according to what is described here below for the module concerned. Such a software component is executed by a data processor of a physical entity (terminal, server, gateway, router etc) and is capable of accessing hardware resources of this physical entity (memories, recording media, communications buses, input/output electronic boards, user interfaces etc).

In the same way, a hardware component corresponds to any element of a hardware assembly capable of implementing a function or a set of functions according to what is described here below for the module concerned. It can be a programmable hardware component or a component with an integrated processor for the execution of software, for example, an integrated circuit, a smart card, a memory card, an electronic board for the execution of firmware etc.

Each component of the system described here above implements of course its own software modules.

The different embodiments mentioned here above can be combined with one another to implement the invention.

Referring to figure 4, we present a simplified architecture of a device capable of implementing the described technique. Such a device comprises a memory 41, a processing unit 42 equipped for example with a microprocessor and driven by the computer program 43 implementing at least one part of the method as described. In at least one embodiment, the invention is implemented in the form of an application installed on a scheduling device. Such a device comprises the necessary means for implementing the proposed technique as described herein before. According to the disclosure, the device may be an independent device connected to an EEG recording and processing device or directly being integrated in a EEG recording and processing device.

**Claims**

1.  A method of constructing a value representative of an interaction between a plurality of brain regions, the method being implemented by an electronic device, said electronic device comprising a processor and a memory, **characterized in that** it comprises:

- obtaining (10), in the form of connectivity matrices, dynamic functional networks, which are representative of electrical signals measured for a predetermined number of points of interest, called nodes, within a cerebral cortex during a given time period;
- determining (20), from at least one of said connectivity matrices, a global efficiency (GE) score and at least one clustering coefficient score (Cc) for each node of said at least one of said connectivity matrices;
- calculating (30) a value representative of an interaction between the plurality of brain networks in the form of a distribution ratio using said global efficiency score and said clustering coefficient scores (Cc).

2. The method according to claim 1, wherein determining said global efficiency (GE) score comprises calculating:

$$GE = \frac{1}{N} \sum_{i}^{N} E_i$$

Where $E_i$ is the efficiency of each node I computed through the shortest path lengths between nodes.

3. The method according to claim 1, wherein determining one clustering coefficient score (Cc) of one node comprises calculating:

$$Cc(i) = \frac{2L_i}{k_i(k_i - 1)}$$

Where L represents the number of links between the $k_i$ neighbors of node i.

4. The method according to claim 1, **characterized in that** calculating (30) comprises, for at least one connectivity matrix associated to at least one dynamic functional network:

$$DI = \frac{GE}{\sum_{i}^{N} Cc}$$

where GE is the global efficiency of the network, Cc is the clustering coefficient and N is the number of nodes in the network.

5. The method according to claim 2, **characterized in that** N is equal to 68.

6. The method according to claim 1, **characterized in**

**that** obtaining (10) connectivity matrices comprises:

- obtaining (10-1) signals representing of a cerebral activity for a given period of time;
- constructing (10-2), using the previously obtained signals, a plurality of data structures representative of the functional connectivity between a plurality of regions of interest for a given frequency;
- identifying (10-3), within said plurality of functional connectivity data structures, dynamic functional networks;

7. The method according to claim 1, **characterized in that** determining (20) comprises, for a given connectivity matrix:

- calculating (20-1) the global efficiency (GE) score;
- calculating (20-2) an individual clustering coefficient score (Cc) for each node of said connectivity matrix.

8. An electronic device for obtaining a value representative of an interaction between a plurality of brain networks, the electronic device comprising a processor and a memory, **characterized in that** the device comprises the necessary means for:

- obtaining, in the form of connectivity matrices, dynamic functional networks, which are representative of electrical signals measured for a predetermined number of points of interest, called nodes, within a cerebral cortex during a given time period;
- determining, from at least one of said connectivity matrices, a global efficiency (GE) score and at least one clustering coefficient score (Cc) for each node of said at least one of said connectivity matrices;
- calculating a value representative of an interaction between the plurality of brain networks in the form of a distribution ratio using said global efficiency score and said clustering coefficient scores (Cc).

9. A Computer program product downloadable from a communication network and/or stored on a computer-readable support and/or executable by a computer **characterized in that** it comprises program code instructions for the execution of a method according to claim 1, when it is executed on a processor.

Figure 1

Figure 2

Figure 3

Figure 4

Europäisches
Patentamt

European
Patent Office

Office européen
des brevets

## EUROPEAN SEARCH REPORT

Application Number

EP 18 19 7414

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YUAN-YUAN QIN ET AL: "Frequency-specific Alterations of Large-scale Functional Brain Networks in Patients with Alzheimer's Disease", CHINESE MEDICAL JOURNAL / ZHONGHUA YIXUE ZAZHI YINGWEN BAN., vol. 128, no. 5, 5 March 2015 (2015-03-05), pages 602-609, XP055565305, CN ISSN: 0366-6999, DOI: 10.4103/0366-6999.151654 * abstract * * section "Construction of brain functional network" * * section "Topological parameter comparisons at different frequency bands" * ----- | 1-9 | INV. G16H50/30 |
| X | DANIELLE S BASSETT ET AL: "Human brain networks in health and disease", CURRENT OPINION IN NEUROLOGY, vol. 22, no. 4, 1 August 2009 (2009-08-01), pages 340-347, XP055565326, GB ISSN: 1350-7540, DOI: 10.1097/WCO.0b013e32832d93dd * section "Graph theoretical concepts for network analysis" * ----- | 1-9 | |

TECHNICAL FIELDS
SEARCHED (IPC)

G16H

The present search report has been drawn up for all claims

| Place of search | Date of completion of the search | Examiner |
|---|---|---|
| Munich | 22 March 2019 | Reinbold, Bernhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)